# EUROPEAN PATENT SPECIFICATION

(11) **EP 3 015 099 B1**
(45) Date of publication and mention of the grant of the patent: **17.06.2020**
(21) Application number: 14817440.2
(22) Date of filing: 27.06.2014
(51) Int. Cl.: A61K 8/04, A61K 8/22, A61K 8/34, A61K 8/86, A61Q 5/08, A61Q 5/10, B65D 83/14, B65D 83/62, B65D 83/68

(54) **HAIR COSMETIC MATERIAL, AND HAIR COSMETIC PRODUCT**
HAARKOSMETISCHE MATERIALZUSAMMENSETZUNG UND HAARKOSMETIKPRODUKT
COMPOSITION DE SUBSTANCE COSMÉTIQUE CAPILLAIRE ET PRODUIT COSMÉTIQUE CAPILLAIRE

(30) Priority: 28.06.2013 JP 2013136475; 28.06.2013 JP 2013136476; 02.07.2013 JP 2013139321; 05.07.2013 JP 2013142176
(43) Date of publication of application: 04.05.2016
(73) Proprietor: Hoyu Co., Ltd., Nagoya-shi, Aichi 461-8650 (JP)
(72) Inventor: KONNO, Yoshihiro, Nagakute-shi Aichi 480-1136 (JP); KANAYAMA, Shinya, Nagakute-shi Aichi 480-1136 (JP); KOMODA, Takeshi, Nagakute-shi Aichi 480-1136 (JP)
(74) Representative: TBK
(86) International application number: PCT/JP2014/067208
(87) International publication number: WO 2014/208735

(56) References cited:
- EP-A1- 1 547 937
- FR-A1- 2 221 189
- JP-A- 2001 122 364
- JP-A- 2004 161 292
- JP-A- 2005 194 207
- JP-A- 2012 180 318

## Description

### Technical Field

The invention disclosed in the present application relates to a hair cosmetic material and a hair cosmetic material product as defined in the claims.

### Background Art

A first agent containing an alkali agent and a second agent containing an oxidizing agent are mixed at the time of use. In a so-called aerosol-type hair cosmetic material product utilizing a propellant, a first agent and a second agent of a hair cosmetic material composition are separately filled in an aerosol container and discharged from the aerosol container at the time of use.

The aerosol container in which the first agent, the second agent, and the propellant are filled in separate spaces is a double structure container. In the double structure container, the propellant provides a pressure for discharging the first agent filled in a first inner container and the second agent filled in a second inner container, and the inner containers cause elastic deformation due to the pressure, whereby a filled material becomes possible to be discharged in a cream state as it is. A compressed gas and a liquefied gas can be used as the propellant.

The following PTL 1 discloses a so-called "duplex can" type aerosol container (see PTL 1). In one specific example of this aerosol container, a propellant that is the compressed gas and a first agent or a second agent are filled in a first container and a second container, respectively, and it is possible to discharge each of the first agent and the second agent in a cream state as it is. As is clear from the structure of this aerosol container, the first container and the second container may be different from each other in terms of a propellant amount/pressure in the container. Since the propellant amount/pressure in the container can be freely set in each container, such an aerosol container is liable to cope with the case where the first agent and the second agent have different viscosity/viscousness from each other, or oxygen generated from an oxidizing agent (especially hydrogen peroxide) as the second agent.

The following PTL 2 discloses an aerosol container which can be used in a hair cosmetic material composition. The following PTL 2 discloses a double structure container in which a first inner container and a second inner container are existent in the same outer container.

The following PTL 3 discloses a double structure aerosol container in which a first inner container for filling a first undiluted solution and a second inner container for filling a second undiluted solution are accommodated in the same outer container.

The following PTL 4 discloses a second agent that is said to be excellent in mixing properties with a first agent, not sticky at the time of application to the hair, excellent in extensibility, good in compatibility with a hair, excellent in a bleaching power, uniform in an emulsified state at the time of preparation, and appropriate in viscosity.

There is proposed a double structure container provided with such a mechanism that not only a first agent and a second agent of a hair cosmetic material are separately filled, but also the both agents are simultaneously discharged from the same pressure discharge system (hereinafter referred to as "separate filling/same pressure discharge-type double structure container"). As the separate filling/same pressure discharge-type double structure container, for example, those having the following mechanisms can be exemplified.

That is, an opening of the outer container in which the propellant that is a compressed gas or liquefied gas for pressurization is filled therein (propellant filling space) is airtightly closed by a lid provided with a discharge passage and a valve for opening and closing this. In addition, two bag-like bodies that are inner containers in which the first agent and the second agent of the hair cosmetic material are filled, respectively (a space for filling the first agent and a space for filling the second agent) are placed in the inside of the outer container, and openings of these bag-like bodies are communicated in a liquid-tight manner with the discharge passage of the lid. In consequence, the first agent and the second agent filled in the bag-like bodies always receive a discharge pressure by the same propellant, and the simultaneous discharge of the first agent and the second agent can be controlled by a simple opening and closing operation of a discharge passage valve.

For example, the following PTLs 3 and 5 disclose a double structure container basically provided with such a mechanism.

### Citation List

### Patent Literature

PTL 1: JP-A-2002-240873
PTL 2: JP-A-2001-122364
PTL 3: JP-A-2012-229318
PTL 4: JP-A-2007-217293
PTL 5: JP-A-2013-043659
PTL 6: JP-A-2010-235578
PTL 7: JP-A-2007-314442

JP 2005 194207 A describes a two-pack type hair cosmetic which comprises a first agent comprising an aqueous medium and 0.01 to 3 wt. % of ammonia contained in the aqueous medium and having a viscosity of ≥1,000 mPa s at 25°C, and a second agent comprising an aqueous medium and hydrogen peroxide contained in the aqueous medium. The first agent and the second agent are separately received in the resin-made flexible containers, respectively, stored in the outer can of the aerosol container, and are simultaneously discharged.

JP 2001 122364 A describes a double-walled aerosol container comprising an outer container having an opening segment, a valve segment fixed to the outer container so as to close the opening segment, and an inner bag fixed to the valve segment. Acid hair dye is stored in the inner bag 8 as stored content. The outer container is formed by polyethylene terephthalate and the entire outer container is transparent. In turn, the inner bag is formed into a pleat shape. As acid hair dye is discharged out of the inner bag, the pleated segments are gathered together, shrunk and deformed.

JP 2012 180318 A describes a hair cosmetic composition of an emulsion containing (A) a higher alcohol and (B) a nonionic surfactant, and satisfying the following conditions (a) to (c): (a) the viscosity is 8,000-25,000 mPa s; (b) the formulated percentage of the component (A) is 4.0-8.0 mass%; and (c) the mass ratio of the ≥22C higher alcohol to the whole amount of the component (A) is 0.50-1.

FR 2 221 189 A1 describes the application of a gel to the surface of a substrate, by simultaneously applying first and second liquid streams supplied from separate chambers of a gelable liquid and a complementary gelling agent respectively, the two streams passing along a common flow path to this surface to mix and form a gel.

JP 2004 161292 A describes a package product for delivering a plurality of contents includes an aerosol container, which has an outer container, an inner bag housed in the outer container and having two parallelly partitioned storing parts, and a valve interposed between the inner bag and a delivery hole of the outer container. The package product also includes different types of contents (a first agent A and a second agent B) filled in the storing parts of the inner bag, respectively, and a compressed gas filled in a space between the inner bag and the outer container.

EP 1 547 937 A1 describes an aerosol container provided with an outer container, a collapsible inner bag inserted in the outer container, and a valve. The inner bag comprises an upper and lower chamber, and the chambers are divided at a constriction part formed in the middle of the inner bag by a partitioning member. The valve has communicating holes that communicates valve with the upper chamber and the lower chamber through a dip tube.

### Summary of Invention

### Technical Problem

In the double structure container disclosed in the foregoing PTL 2, since the first inner container and the second inner container are existent in the same outer container, the first agent and the second agent are placed under the same pressure. Therefore, the first agent and the second agent cannot be placed under a different pressure from each other as in the duplex can-type aerosol container. Moreover, there is involved such a problem that after storing the aerosol-type hair cosmetic material product for a certain period of time, it is desired to discharge the first agent and the second agent in a desired ratio.

However, the double structure container has such advantages that the number of constituent components is small; and that it can be produced at low costs. In addition, the double structure container has such an advantage that a degree of freedom for design with respect to the shape of the outer container is high.

In the duplex can-type aerosol container, two double structure containers stand in line and are formed in a wide shape, and hence, from the viewpoint of easiness in grasping the aerosol container, there was room for improvement. The double structure container can be made easy for grasping by forming the outer container in an approximately columnar shape.

Taking the foregoing into consideration, the inventors of the present application determined to adopt a double structure container.

Commercially available duplex can-type inner containers are formed in a cylindrical shape. When the discharge of filled materials is continued, the right and left of the inner container cramp up, and the inner container is not crushed tightly. Therefore, though flow passages of the filled materials in the inner container are ensured, residual amounts of the filled materials which cannot be discharged become large.

However, while hydrogen peroxide that is an oxidizing agent is excellent in a bleaching power of melamine in the hair, there is room for improvement in storage stability. In particular, the hydrogen peroxide becomes instable under irradiation with sunlight.

There is also involved such a defect that when the hydrogen peroxide is decomposed, not only its oxidizing power is lowered, but also generated oxygen moves into the propellant filling space. In addition, when the oxygen generated by decomposition of hydrogen peroxide resides in the second inner container, on the occasion of discharging the second agent from the double structure container, there is a possibility that the oxygen is released under atmospheric pressure simultaneously with the second agent.

In the foregoing PTL 4, the uniformity of emulsion and the appropriateness of viscosity at the time of preparation of the second agent are evaluated. However, in the foregoing PTL 4, the storage of the second agent under irradiation with sunlight is not discussed at all.

In the double structure container in which the first inner container and the second inner container are existent in the same outer container, if the outer container and the second inner container are constituted to include a light-permeable material, thereby making the residual amount of the second agent in the second container visible, the case where the second agent is placed under irradiation with sunlight is assumed, and an enhancement of the stability of hydrogen peroxide becomes important.

Now, in view of the mechanism of the above-described separate filling/same pressure discharge-type double structure container, it is necessary to robustly constitute the outer container having a propellant sealed therein. In addition, if the reduced states of the first agent and the second agent filled in the bag-like bodies are viewable from the outside, such is convenient for a user, and hence, the outer container is constituted of, for example, a hard and transparent plastic material. Meanwhile, for the bag-like bodies for filling the first agent and the second agent, it is necessary to use a material that is relatively soft and readily deformable by pressurization such that the contents are surely discharged by a pressure of the propellant for pressurization.

In consequence, in many cases, the outer container and the bag-like bodies differ from each other in terms of impact resistance strength. For that reason, when the double structure container receives a large impact, for example, in the case where a double structure container is dropped from a hand during the use and collided on a hard floor surface, there may be a possibility that the bag-like bodies filled with the first agent and the second agent, respectively are broken. In addition, the liquid tightness (seal) in a connection portion between the opening of the above-described bag-like body and the discharge passage of the lid becomes loose, resulting in a possibility that the first agent or second agent filled in the bag-like body leaks out.

In a hair cosmetic material, such as an oxidation hair dyeing agent or a hair bleaching agent, by compounding an alkali agent and an oxidizing agent in the first agent and the second agent, respectively and uniformly mixing the both agents at the time of application to the hair, a hair dyeing effect or a hair bleaching effect is increased. Then, on the occasion of mixing the first agent and the second agent, a reactive gas, such as an oxygen gas (O₂), carbon dioxide, an ammonia gas, etc., is generated. In the case where the first agent and the second agent leak out due to the breakage of the above-described bag-like bodies, the both agents come into contact with each other to cause mixing to some extent, too. In consequence, the reactive gas is generated in the inside in the outer container, the amount of which is, however, small as compared with the case of artificially uniformly mixing the both agents at the time of application to the hair.

A yet another aspect of the present invention is to inhibit the generation of a reactive gas, such as an oxygen gas, etc., even when under special conditions that the first agent and the second agent leak out in the inside of the outer container from the bag-like bodies of the above-described double structure container, the first agent and the second agent come into contact with each other.

In a process of pursuing means for solving the foregoing problems, the inventors of the present application have obtained the following three findings.
(1) With respect to the generation of a reactive gas due to "intermixing" of the first agent and the second agent, it is important to consider intermixing of the both agents in a macroscopic meaning and intermixing in a microscopic meaning, namely mutual invasion or diffusion on the contact boundary between the both agents.
   It is to be noted that the "intermixing" of the first agent and the second agent as referred to in the specification of the present application refers to both meanings including the intermixing in a macroscopic meaning and the intermixing in a microscopic meaning as described above, unless otherwise specifically indicated.
(2) In inhibiting the above-described intermixing, it is effective to regulate the viscosity of each of the first agent and the second agent within a certain range, and in order to achieve this, it is effective to control the content of the surfactant, the oily component, or the higher alcohol in the both agents.
(3) For the purpose of solving the problem under special conditions for "preventing an increase of a gas internal pressure of a small-capacity outer container", the foregoing means are useful; however, its effect for inhibiting mixing properties is an extent of not giving an influence so much against artificial uniform mixing of the first agent and the second agent at the time of application to the hair.

Now, the separate filling/same pressure discharge-type double structure container is generally designed so as to simultaneously discharge the same amounts of the first agent and the second agent filled in the bag-like bodies. The first agent and the second agent to be filled therein are also discharged in the same amounts in a mass ratio of 1/1 and then mixed, followed by application to the hair.

However, the separate filling/same pressure discharge-type double structure container has a mechanism so as to simultaneously discharge the first agent and the second agent by the same discharge pressure of the propellant. Therefore, in particular, in the case where the first agent and the second agent of the hair cosmetic material are a creamy preparation having a relatively high viscosity, unless properly regulating rheology properties (discharge properties by flowing at the time of pressurization) of each of the first agent and the second agent, actually, the equal amount discharge of the first agent and the second agent cannot be realized. In the case where the equal amount discharge properties are impaired to some degree or more, a commercial value itself of the hair cosmetic material product in which the first agent and the second agent are filled in the double structure container is affected.

Furthermore, in the case where the hair cosmetic material is, for example, a hair dyeing agent, such as a two-agent type oxidation hair dyeing agent or hair bleaching agent, etc., at least an alkali agent (furthermore, an oxidation dye) is compounded in the first agent, and an oxidizing agent is compounded in the second agent. Under such a restriction in view of composition, in order to achieve the equal amount discharge, it is not easy to decide what kind of category of rheology properties to be focused on and to grasp realization of the rheology properties through what kind of composition design of the first agent and the second agent.

The foregoing PTLs 6 and 7 are aimed to realize rheology properties with respect to a cosmetic material composition. However, PTL 6 discloses that with respect to a two-agent type hair dyeing agent to be discharged in a foam state from an aerosol foamer container, the dynamic viscoelasticity of a foam after discharge and mixing is regulated from the viewpoints of permeability after application to the hair, prevention of dripping from the hair, and the like. In addition, PTL 7 discloses that on review of spreading of a skin cosmetic material onto a skin at the time of application, or the like, an average emulsion particle diameter in an oil-in-water emulsified cosmetic material is regulated, thereby providing the cosmetic material with certain rheology properties. In consequence, PTLs 6 and 7 are not a good guide at all to realization of the above-described equal amount discharge in view of not only category of rheology properties but also composition design of the first agent and the second agent for achieving that.

A further aspect of the present invention is to provide a hair cosmetic material capable of performing equal amount discharge of a first agent and a second agent by a separate filling/same pressure discharge-type double structure container and provided with rheology properties with which the equal amount discharge properties can be kept with time.

### Solution to Problem and Advantageous Effects of Invention

The invention of the present application is concerned with a hair cosmetic material including a first agent containing an alkali agent and a second agent containing an oxidizing agent. The first agent and the second agent are used in a double structure container provided with a mechanism of separating the first agent and the second agent from each other and simultaneously discharging the both agents by the above-described propellant. In this double structure container, a space for filling the first agent and a space for filling the second agent are each independently provided in the inside of a propellant filling space having a propellant for pressurization filled therein. The first agent and the second agent are each filled in the space for filling the respective agents.

Each of the first agent and the second agent is discharged in a liquid state; the first agent contains an alkali agent, whereas the second agent contains an oxidizing agent; and each of the first agent and the second agent has a viscosity falling within the range of from 7,000 to 30,000 mPa·s at 25°C.

Such a hair cosmetic material includes a first agent and a second agent, and the first agent contains an alkali agent, whereas the second agent contains an oxidizing agent. Then, the first agent and the second agent are respectively filled in a space for filling the first agent and a space for filling the second agent (for example, two bag-like bodies), each of which is independently provided, in a propellant filling space (inner space of the outer container) of the double structure container. In consequence, if a strong impact is applied to the double structure container, the generation of a reactive gas, such as an oxygen gas, etc., may possibly occur due to the contact between the first agent and the second agent, each of which has leaked out into the propellant filling space from each of the space for filling the first agent and the space for filling the second agent.

However, on that occasion, it has been noted that when the viscosity of each of the first agent and the second agent is designed to be 7,000 mPa·s or more at 25°C, the generation of a reactive gas which causes a gas internal pressure in the propellant filling space to be excessively increased is effectively inhibited. A reason for this may be considered to reside in the matter that if the viscosity of each of the first agent and the second agent is 7,000 mPa·s or more, the intermixing of the first agent and the second agent is inhibited, whereby a reactive gas is not generated to such an extent that the gas internal pressure in the propellant filling space is excessively increased. Moreover, in this case, an effect for inhibiting the intermixing of the first agent and the second agent is in such an extent that artificial uniform mixing of the first agent and the second agent at the time of application to the hair is not so much affected.

From the standpoint of ensuring the foregoing effect, an upper limit value of the viscosity of each of the first agent and the second agent at 25°C is not particularly limited. However, from the points of view of achieving smooth discharge of the first agent and the second agent from the double structure container and making artificial mixing after discharge and before application to the hair easier, the upper limit value of the viscosity of each of the first agent and the second agent is set to 30,000 mPa·s.

In such a hair cosmetic material, each of the first agent and the second agent may contain at least one surfactant, and a content of the surfactant of each of the agents may be 10% by mass or less.

When the first agent and the second agent leak out, as factors of controlling the easiness of intermixing of the both agents, in addition to the viscosities, surface tensions of the both agents are exemplified. If the surface tensions are low, the first agent and the second agent are easily intermixed with each other. The content of the surfactant in each of the first agent and the second agent of the hair cosmetic material is 10% by mass or less. In consequence, lowerings of the surface tensions of the both agents are suppressed, and the first agent and the second agent, each of which has leaked out into the propellant filling space, are hardly intermixed with each other, and hence, the generation of an oxygen gas is inhibited. This effect does not affect so much the artificial uniform mixing of the first agent and the second agent at the time of application to the hair.

From the standpoint of ensuring such an effect, a lower limit value of the content of each of the surfactants in the first agent and the second agent is not particularly limited. However, from the point of view of emulsion stability, it is preferred that the content of the surfactant in each of the both agents is, for example, 1.5% by mass or more.

In addition, in such a hair cosmetic material, each of the first agent and the second agent may contain at least one oily component, and a total content of the oily components in the first agent and the second agent relative to a total amount of the first agent and the second agent may be 10% by mass or less.

It is known that the surface tension of the composition is lowered by the oily component. The total content of the oily components contained in the first agent and the second agent of the hair cosmetic material is 10% by mass or less relative to the total amount of the first agent and the second agent. For this reason, lowerings of the surface tensions of the both agents are suppressed, and the first agent and the second agent, each of which has leaked out into the propellant filling space, are hardly intermixed with each other, and hence, the generation of an oxygen gas is inhibited. This effect does not affect so much the artificial uniform mixing of the first agent and the second agent at the time of application to the hair.

Furthermore, in such a hair cosmetic material, each of the first agent and the second agent may contain at least one higher alcohol, and a total value of the following higher alcohol indexes regarding the higher alcohol contained in each of the agents may be 140 or less.

The higher alcohol index as referred to herein is an integrated value (a × b) of a carbon number (a) of the higher alcohol and a content value (b) in the first agent or the second agent of the higher alcohol in terms of a mass% unit. This higher alcohol is a monohydric alcohol having 12 or more and 22 or less carbon atoms, which is a linear or branched, saturated or unsaturated alcohol.

Although the higher alcohol may be generally considered to be one kind of oily components, in the present invention, the higher alcohol and the oily component are distinguished from each other. The content of the higher alcohol influences the surface tension of the composition, and such an influence also varies with the carbon number (molecular weight) of the higher alcohol. Then, the inventors of the present application thought a concept of "higher alcohol index" as an index of evaluating the influence of the higher alcohol against the inhibition of the generation of an oxygen gas (inhibition of lowerings of the surface tensions of the first agent and the second agent).

The total value of the higher alcohol indexes regarding the higher alcohol to be contained in each of the first agent and the second agent of the hair cosmetic material is 140 or less. For this reason, lowerings of the surface tensions of the first agent and the second agent are suppressed. Therefore, the first agent and the second agent, each of which has leaked out into the propellant filling space, are hardly intermixed with each other, and hence, the generation of an oxygen gas is inhibited. This effect does not affect so much the artificial uniform mixing of the first agent and the second agent at the time of application to the hair.

In addition, a hair cosmetic material product which is constituted to include such a hair cosmetic material and a double structure container provided with a mechanism of separating the first agent and the second agent from each other and simultaneously discharging the both agents by the above-described propellant may also be provided. In this double structure container, a space for filling the first agent and a space for filling the second agent are each independently provided in the inside of a propellant filling space having a propellant for pressurization filled therein. The first agent and the second agent of the hair cosmetic material are each filled in the space for filling the respective agents.

It is to be noted that in the case where the hair cosmetic material is constituted to include a third agent in a powder state or the like, the hair cosmetic material product may also include, in addition to the double structure container having the first agent and the second agent filled therein, the third agent attached thereto.

A hair cosmetic material product in which a first agent and a second agent of a hair cosmetic material are filled in a space for filling the first agent and a space for filling the second agent, respectively in a double structure container is provided. In this hair cosmetic material product, the generation of an oxygen gas due to the contact between the first agent and the second agent, which have leaked out from the space for filling the first agent and the space for filling the second agent, respectively in the double structure container, is effectively inhibited.

### Brief Description of Drawings

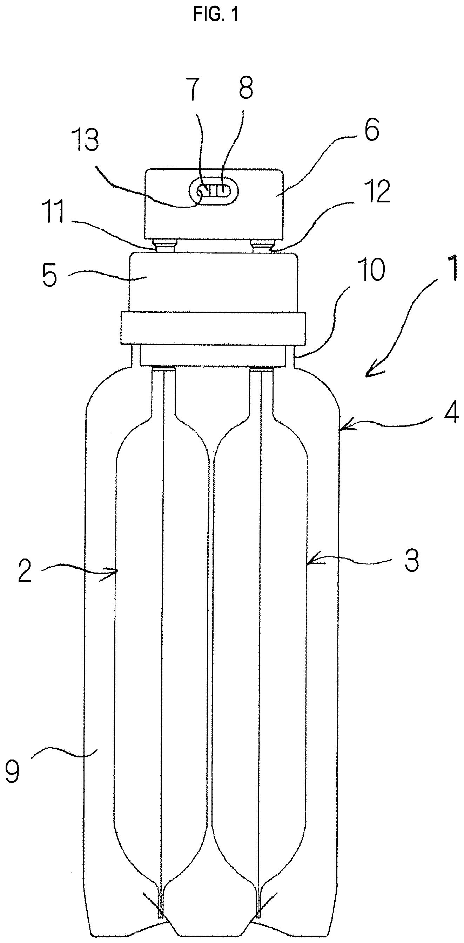

[Fig. 1] Fig. 1 is a front view showing an example of a double structure container of the present invention.

### Reference Signs List

- 1:: Double structure container
- 2:: First inner container
- 3:: Second inner container
- 4:: Outer container
- 5:: Valve unit
- 6:: Actuator
- 7, 8:: Discharge hole
- 9:: Propellant filling space
- 10:: Opening
- 11, 12:: Cylindrical stem
- 13:: Opening

### Description of Embodiments

The inventions as disclosed in the present application are hereunder explained.

### [Hair cosmetic material]

First of all, the hair cosmetic material of the present invention is explained centering on a first agent and a second agent. Details of main components mentioned in this embodiment are described later.

The hair cosmetic material of the present invention is constituted to include at least a first agent containing an alkali agent and a second agent containing an oxidizing agent. These first agent and second agent are respectively filled in a space for filling the first agent and a space for filling the second agent, each of which is, for example, a bag-like body, in a separate filling/same pressure discharge-type double structure container as described later. Each of the first agent and the second agent of the hair cosmetic material is a liquid dosage form and is discharged as a liquid from the double structure container. Although the contents of the "liquid dosage form" are not always limited, examples thereof include a cream, a gel, a milky lotion, and the like. Of those, a cream and a gel, in which a relatively high viscosity is liable to be ensured, are preferred.

As the hair cosmetic material, such a two-agent type composed of the first agent and the second agent is exemplified; however, a multi-agent type such as a three-agent type, in which a third agent or the like according to an appropriate preparation is further added, is also included. The third agent or the like may be a liquid or may be a powder or the like. In the case where the hair cosmetic material is a three-agent type or the like, in general, the third agent or the like is attached to the double structure container having the first agent and the second agent filled therein, whereby it becomes a constituent element of a hair cosmetic material product as a commodity.

Examples of a category of the hair cosmetic material include an oxidation hair dyeing agent, a hair bleaching agent, and a hair dedyeing agent. Although these are common from the standpoint of including the first agent containing an alkali agent and the second agent containing an oxidizing agent, the oxidation hair dyeing agent further includes an oxidation dye. The oxidation dye is composed of a principal intermediate, or composed of a principal intermediate and a coupler; however, as the case may be, a direct dye is further added. In the hair dedyeing agent, a persulfate is added as an oxidation aid in addition to the alkali agent.

In the hair cosmetic material of the present invention, a viscosity of each of the first agent and the second agent falls within the range of from 7,000 to 30,000 mPa·s at 25°C and more preferably falls within the range of from 10,000 to 25,000 mPa·s.

This viscosity can be, for example, measured by using a B-type viscometer for one minute at a rotating rate of 12 rpm/min under conditions of using a No. 4 rotor. As a specific example of the B-type viscometer, for example, a BL-type viscometer, VISCOMETER (available from Toki Sangyo Co., Ltd.) can be exemplified.

Each of the first agent and the second agent of the hair cosmetic material may not contain a propellant for foaming, or may contain a propellant for foaming. In the case where each of the first agent and the second agent contains a propellant for foaming, the hair cosmetic material is corresponding to the category of an aerosol-type foam hair cosmetic material. As the propellant for foaming, liquefied gases, such as LPG, dimethyl ether, isopentane, etc., and compressed gases, such as carbon dioxide, a nitrogen gas, etc., can be exemplified; however, in particular, liquefied gases are preferred. A mass ratio of a neat liquid of the first agent or the second agent (a composition in a state of not containing a propellant) to the propellant preferably falls within the range of from 90/10 to 98/2.

Even in the hair cosmetic material not containing a propellant for foaming, the hair cosmetic material may also be a hair cosmetic material in which either one agent of the first agent and the second agent contains an organic acid, for example, citric acid, etc., the other agent contains a carbonate such as sodium carbonate, or a hydrogencarbonate such as sodium hydrogencarbonate, and these agents are mixed to form a foam.

Next, each of the first agent and the second agent of the hair cosmetic material can contain a surfactant. As for the kind of the surfactant, any of a cationic surfactant, an anionic surfactant, an ampholytic surfactant, or a nonionic surfactant may be used, and these may also be arbitrarily combined. However, the case where when the first agent contains an anionic surfactant, then the second agent contains a cationic surfactant; or conversely, when the first agent contains a cationic surfactant, then the second agent contains an anionic surfactant, is preferred from the standpoint of making it easier to achieve uniform mixing after discharging the first agent and the second agent. Although a content of the surfactant in each of the first agent and the second agent is not limited, it is preferably 10% by mass or less in each case, and more preferably in the range of from 2.5 to 8% by mass in each case.

Each of the first agent and the second agent of the hair cosmetic material can contain an oily component. Although a content of the oily component in each of the first agent and the second agent is not limited, it is preferably 10% by mass or less, and more preferably 8% by mass or less in each case. As the oily component, hydrocarbons or esters are especially preferred.

Furthermore, when a relation between the content of the oily component of the first agent (former) and the content of the oily component of the second agent (latter) is allowed to fall within the range of 1.05 to 5, and especially within the range of 1.1 to 3 in terms of a mass% unit of the former to the latter, an oily feeling is different between the former and the latter, and hence, such is preferred from the standpoint of inhibiting the intermixing of the first agent and the second agent, each of which has leaked out into a propellant filling space.

Next, the first agent and/or the second agent of the hair cosmetic material can contain a higher alcohol. A higher alcohol having a carbon number in the range of from 12 to 22 is especially preferred. A preferred content of the higher alcohol to be contained in each of the first agent and the second agent can be mentioned by a higher alcohol index that is an integrated value (a × b) of a carbon number (a) of the higher alcohol and a content value (b) in the first agent or the second agent of the higher alcohol in terms of a mass% unit. That is, a total value of the higher alcohol indexes regarding the higher alcohol contained in each of the first agent and the second agent is preferably 140 or less in each case, and more preferably in the range of from 40 to 130 in each case.

Furthermore, when a relation between a total value of the higher alcohol indexes of the first agent (former) and a total value of the higher alcohol indexes of the second agent (latter) is allowed to fall within the range of 1.05 to 5, and especially within the range of 1.1 to 3 in terms of a mass% unit of the former to the latter, an oily feeling is different between the former and the latter, and hence, such is preferred from the standpoint of inhibiting the intermixing of the first agent and the second agent, each of which has leaked out into a compressed gas filling space.

In addition to the foregoing points, it is also preferred that a higher alcohol having 16 or less carbon atoms accounts for 50% by mass or more of the higher alcohol to be compounded in either one agent of the first agent and the second agents, whereas a higher alcohol having 18 or more carbon atoms accounts for 50% by mass or more of the higher alcohol to be compounded in the other agent. In this case, since an oily feeling is different between the first agent and the second agent, though mixing in such a state that an artificial external force does not act, such as a state of intermixing of the first agent and the second agent, each of which has leaked out into a propellant filling space, hardly occurs, mixing in such a state that an artificial external force acts, such as a state of mixing after discharge of the first agent and the second agent (for example, mixing/application by a brush), is easy.

### [Principal components of hair cosmetic material]

Next, embodiments of the essential components and the principal arbitrary compounding components to be contained in the hair cosmetic material of the present invention are successively described in detail.

### (Alkali agent)

In the case where the hair cosmetic material is an oxidation hair dyeing agent, a hair bleaching agent, or a hair dedyeing agent, examples of the alkali agent to be contained in the first agent include ammonia, alkanolamines, silicates, carbonates, hydrogencarbonates, metasilicates, sulfates, chlorides, phosphates, basic amino acids, and the like. Specifically, examples of the alkanolamine include monoethanolamine, triethanolamine, and the like; examples of the silicate include sodium silicate and potassium silicate; examples of the carbonate include sodium carbonate and ammonium carbonate; examples of hydrogencarbonate include sodium hydrogencarbonate and ammonium hydrogencarbonate; examples of the metasilicate include sodium metasilicate and potassium metasilicate; examples of the sulfate include ammonium sulfate; examples of the chloride include ammonium chloride; examples of the phosphate include monobasic ammonium phosphate and dibasic ammonium phosphate; and examples of the basic amino acid include arginine, lysine, and salts thereof. Of these, ammonia, carbonates, and ammonium salts are preferred.

Although a content of the alkali agent in the first agent is not limited, it is, for example, 0.1 to 15% by mass, and more preferably 1 to 10% by mass.

### (Oxidizing agent and oxidation aid)

In the case where the hair cosmetic material is an oxidation hair dyeing agent, a hair bleaching agent, or a hair dedyeing agent, examples of the oxidizing agent to be contained in the second agent include hydrogen peroxide, urea peroxide, melamine peroxide, sodium percarbonate, potassium percarbonate, sodium perborate, potassium perborate, ammonium persulfate, sodium peroxide, potassium peroxide, magnesium peroxide, barium peroxide, calcium peroxide, strontium peroxide, hydrogen peroxide adducts of sulfates, hydrogen peroxide adducts of phosphates, hydrogen peroxide adducts of pyrophosphates, and the like. Of these, hydrogen peroxide is preferred.

Although a content of the oxidizing agent in the second agent is not particularly limited, it is, for example, 0.1 to 15% by mass, and more preferably 1 to 10% by mass. In the case where the second agent contains hydrogen peroxide as the oxidizing agent, it is preferred that ethylene glycol phenyl ether (phenoxyethanol), or hydroxyethanediphosphonic acid or a salt thereof is compounded as a stabilizer for enhancing the stability in the acidic agent.

Meanwhile, examples of the oxidation aid include persulfates, such as ammonium persulfate, potassium persulfate, sodium persulfate, etc.

### (Oxidation dye and direct dye)

In the case where the hair cosmetic material is an oxidation hair dyeing agent, among oxidation dyes to be contained in the first agent, the principal intermediate is a dye precursor that is mainly an o- or p-phenylenediamine or an aminophenol, and in general, it is a compound that is colorless or weakly colored itself. The principal intermediate is used alone, or used together with a coupler.

Examples of the principal intermediate include p-phenylenediamine, toluene-2,5-diamine (p-toluylene-diamine), N-phenyl-p-phenylenediamine, 4,4'-diaminodiphenylamine, p-aminophenol, o-aminophenol, p-methylaminophenol, N,N-bis(2-hydroxyethyl)-p-phenylenediamine, 2-hydroxyethyl-p-phenylenediamine, o-chloro-p-phenylenediamine, 4-amino-m-cresol, 2-amino-4-hydroxyethylaminoanisole, 2,4-diaminophenol, and salts thereof, and the like. Examples of the salt include hydrochlorides, sulfates, acetates, and the like.

As the coupler, m-diamines, m-aminophenols, and m-diphenols are mainly exemplified. Specifically, examples thereof include resorcin, catechol, pyrogallol, phloroglucin, gallic acid, hydroquinone, 5-amino-o-cresol, m-aminophenol, 5-(2-hydroxyethylamino)-2-methylphenol, m-phenylenediamine, 2,4-diaminophenoxyethanol, toluene-3,4-diamine, α-naphthol, 2,6-diaminopyridine, diphenylamine, 3,3'-iminodiphenyl, 1,5-dihydroxynaphthalene, tannic acid, and salts thereof, and the like.

Examples of the direct dye which may be additionally used for the purpose of regulating the dyed hair color tone include various acid dyes, basic dyes, nitro dyes, natural dyes, disperse dyes, and HC dyes, and the like.

### (Surfactant)

A surfactant can be contained in the first agent and/or the second agent of the hair cosmetic material. Various cationic, anionic, ampholytic or nonionic surfactants can be used as the surfactant. In all of the first agent and the second agent, a content of the surfactant is preferably 10% by mass or less in each case, and especially preferably in the range of from 2.5 to 8% by mass in each case.

Examples of the cationic surfactant include lauryltrimethylammonium chloride, cetyltrimethylammonium chloride, stearyltrimethylammonium chloride (steartrimonium chloride), behenyltrimethylammonium chloride (behentrimonium chloride), distearyldimethylammonium chloride, cetyltrimethylammonium bromide, stearyltrimethylammonium bromide, an ethyl sulfuric acid lanolin fatty acid aminopropylethyl dimethylammonium, stearyltrimethylammonium saccharinate, cetyltrimethylammonium saccharinate, methacryloyloxyethyltrimethylammonium chloride, behenyltrimethylammonium methyl sulfate, and the like.

Examples of the anionic surfactant include alkyl ether sulfates, polyoxyethylene (hereinafter referred to as "POE") alkyl ether sulfates, alkyl sulfates, alkenyl ether sulfates, alkenyl sulfates, olefin sulfonates, alkane sulfonates, saturated or unsaturated fatty acid salts, alkyl or alkenyl ether carboxylates, α-sulfone fatty acid salts, N-acylamino acid type surfactants, phosphoric mono- or diester type surfactants, and sulfosuccinic acid esters. A counter ion of an anionic group of such a surfactant may be any of a sodium ion, a potassium ion, or triethanolamine.

More specifically, examples of the anionic surfactant include sodium lauryl sulfate, sodium myristyl sulfate, potassium lauryl sulfate, ammonium lauryl sulfate, triethanolamine lauryl sulfate, sodium cetyl sulfate, sodium stearyl sulfate, polyoxyethylene (POE) lauryl ether sodium sulfate, POE lauryl ether triethanolamine sulfate, POE lauryl ether ammonium sulfate, POE stearyl ether sodium sulfate, sodium stearoylmethyltaurate, triethanolamine dodecylbenzenesulfonate, sodium tetradecenesulfonate, sodium lauryl phosphate, POE lauryl ether phosphoric acid and salts thereof, N-lauroyl glutamates (e.g., sodium lauroyl glutamate, etc.), N-lauroylmethyl-β-alanine salts, N-acyl glycine salts, and N-acyl glutamates, as well as lauric acid and myristic acid, each of which is a higher fatty acid, and salts of these higher fatty acids.

Examples of the ampholytic surfactant include alkyl betaine types, fatty acid amide propyl betaine types, alkyl imidazole types, and amino acid types.

More specifically, examples of the ampholytic surfactant include lauryl betaine, imidazoline, amide betaine, carbobetaine, sulfobetaine, hydroxysulfobetaine, amide sulfobetaine, sodium 2-undecyl-N-carboxymethyl-N-hydroxyethyl imidazolinium betaine, cocoamidopropyl betaine, lauryl dimethylaminoacetic acid betaine, stearyl dimethylaminoacetic acid betaine, coconut oil fatty acid amidopropyl betaine, and the like.

Examples of the nonionic surfactant include ether types and ester types.

Specifically, examples of the ether-type nonionic surfactant may include POE cetyl ether (ceteth), POE stearyl ether (steareth), POE behenyl ether, POE oleyl ether (oreth), POE lauryl ether (laureth), POE octyl dodecyl ether, POE hexyl decyl ether, POE isostearyl ether, POE nonyl phenyl ether, and POE octyl phenyl ether.

Specifically, examples of the ester-type nonionic surfactant may include monooleic acid POE sorbitan, monostearic acid POE sorbitan, monopalmitic acid POE sorbitan, monolauric acid POE sorbitan, trioleic acid POE sorbitan, monostearic acid POE glycerin, monomyristic acid POE glycerin, tetraoleic acid POE sorbite, hexastearic acid POE sorbite, monolauric acid POE sorbite, POE sorbite beeswax, monooleic acid polyethylene glycol, monostearic acid polyethylene glycol, monolauric acid polyethylene glycol, lipophilic glyceryl monooleate, lipophilic glyceryl monostearate, self-emulsifying glyceryl monostearate, sorbitan monooleate, sorbitan sesquioleate, sorbitan trioleate, sorbitan monostearate, sorbitan monopalmitate, sorbitan monolaurate, sucrose fatty acid ester, decaglyceryl monolaurate, decaglyceryl monostearate, decaglyceryl monooleate, and decaglyceryl monomyristate.

### (Oily component)

An oily component can be contained in the first agent and/or the second agent of the hair cosmetic material. Examples of the oily component include a fat and oil, a wax, a higher fatty acid, an alkyl glyceryl ether, an ester, a silicone, a hydrocarbon, and the like. In all of the first agent and the second agent, a content of the oily component is preferably 10% by mass or less, and more preferably 8% by mass or less in each case.

Examples of the fat and oil include olive oil, rose hip oil, camellia oil, shea butter, macadamia nut oil, almond oil, tea seed oil, safflower oil, sunflower oil, soybean oil, cottonseed oil, sesame oil, beef tallow, cacao butter, corn oil, peanut oil, rapeseed oil, rice bran oil, rice germ oil, wheat germ oil, *Coix lacryma-jobi* seed oil, grape seed oil, avocado oil, carrot oil, castor oil, linseed oil, coconut oil, mink oil, egg yolk oil, and the like.

Examples of the wax include beeswax, candelilla wax, carnauba wax, jojoba oil, lanolin, spermaceti wax, rice bran wax, sugar cane wax, palm wax, montan wax, cotton wax, bayberry wax, shellac wax, and the like.

Examples of the higher fatty acid include lauric acid, myristic acid, palmitic acid, stearic acid, behenic acid, isostearic acid, hydroxystearic acid, 12-hydroxystearic acid, oleic acid, undecylenic acid, linoleic acid, ricinoleic acid, lanolin fatty acid, and the like.

Examples of the alkyl glyceryl ether include batyl alcohol (monostearyl glyceryl ether), chimyl alcohol (monocetyl glyceryl ether), selachyl alcohol (monooleyl glyceryl ether), isostearyl glyceryl ether, and the like.

Examples of the ester include diisobutyl adipate, cetyl octanoate, isononyl isononanoate, diisopropyl sebacate, octyldodecyl myristate, isopropyl palmitate, stearyl stearate, hexyl laurate, hexyldecyl dimethyloctanoate, triisodecyl myristate, fatty acids (C10-30) (cholesteryl/lanosteryl), lauryl lactate, acetylated lanolin, ethylene glycol di-2-ethylhexanoate, dipentaerythritol fatty acid esters, N-alkyl glycol monoisostearates, diisostearyl malate, and the like.

Examples of the silicone include dimethyl polysiloxane (INCI name: dimethicone), dimethyl polysiloxane having a hydroxyl terminal group (INCI name: dimethiconol), methylphenyl polysiloxane, decamethyl cyclopentasiloxane, a polyether-modified silicone, a highly polymerized silicone having an average polymerization degree of 650 to 10,000, an amino-modified silicone, a betaine-modified silicone, an alkyl-modified silicone, an alkoxy-modified silicone, a carboxy-modified silicone, and the like.

Among the foregoing, examples of the amino-modified silicone include an aminopropylmethylsiloxane-dimethylsiloxane copolymer (INCI name: aminopropyl dimethicone), an aminoethylaminopropylsiloxane-dimethylsiloxane copolymer (INCI name: amodimethicone), an aminoethylaminopropyl-methylsiloxane-dimethylsiloxane copolymer (INCI name: trimethylsilylamodimethicone), and the like.

Examples of the hydrocarbon include an α-olefin oligomer, a light isoparaffin, a light liquid isoparaffin, a liquid isoparaffin, a liquid paraffin, squalane, polybutene, a paraffin, microcrystalline wax, vaseline, and the like.

### (Higher alcohol)

The specified higher alcohol can be contained in the first agent and/or the second agent of the hair cosmetic material. The higher alcohol as referred to herein refers to a monohydric alcohol having 12 or more and 22 or less carbon atoms, which is a linear or branched, saturated or unsaturated alcohol.

A content of the higher alcohol in each of the first agent and the second agent is not always limited. However, when a preferred content thereof is mentioned in terms of the above-described "higher alcohol index", a total value of the higher alcohol indexes, that is an integrated value (a × b) of a carbon number (a) of the higher alcohol and a content value (b) in the first agent or the second agent of the higher alcohol in terms of a mass% unit, is preferably 140 or less, and especially preferably 130 or less in all of the first agent and the second agent.

Specifically, examples of the linear, saturated higher alcohol may include lauryl alcohol, myristyl alcohol, cetyl alcohol (cetanol), stearyl alcohol, arachyl alcohol, and behenyl alcohol. Besides, examples of the branched, saturated higher alcohol may include isostearyl alcohol, 2-hexyldodecanol, 2-octyldodecanol, and the like, and examples of the unsaturated higher alcohol may include oleyl alcohol and the like.

Among the foregoing, lauryl alcohol, myristyl alcohol, cetyl alcohol (cetanol), stearyl alcohol, arachyl alcohol, and behenyl alcohol, all of which are a linear, saturated higher alcohol having a carbon number falling within the range of from 12 to 22, are especially preferred.

### [Other arbitrary compounding components of hair cosmetic material]

In the first agent and/or the second agent of the hair cosmetic material, in addition to the above-described various components, for example, a cationic polymer, a solubilizing agent, a water-soluble polymer compound, a saccharide, an antiseptic, a stabilizer, a pH adjuster, a plant extract, a crude drug extract, a vitamin, a perfume, an antioxidant, an ultraviolet light absorber, a chelating agent, or the like can be arbitrarily compounded. Some of them are hereunder specifically described.

### (Cationic polymer)

Examples of the cationic polymer include cationized cellulose derivatives, polymers or copolymers of diallyl quaternary ammonium salts, and quaternized polyvinylpyrrolidone, and besides, cationic starches, cationized guar gum, and the like.

Examples of the cationized cellulose derivative include a polymer of a quaternary ammonium salt, which is obtained by adding glycidyltrimethylammonium chloride to hydroxyethyl cellulose (polyquaternium-10, for example, LEOGUARD G and LEOGUARD GP, all of which are available from Lion Corporation; and POLYMER JR-125, POLYMER JR-400, POLYMER JR-30M, POLYMER LR-400, and POLYMER LR-30M, all of which are available from Amercho), a hydroxyethyl cellulose/dimethyldiallylammonium chloride copolymer (polyquaternium-4, for example, CELQUAT H-100 and CELQUAT L-200, all of which are available from National Starch and Chemical Corporation), and the like.

Examples of the polymer or copolymer of a diallyl quaternary ammonium salt include a dimethyldiallylammonium chloride polymer (polydimethylmethylene piperidinium chloride) [polyquaternium-6, for example, MERQUAT 100, available from The Lubrizol Corporation], a dimethyldiallylammonium chloride/acrylic acid copolymer [polyquaternium-22, for example, MERQUAT 280, available from The Lubrizol Corporation], an acrylic acid/dially quaternary ammonium salt/acrylamide copolymer [polyquaternium-39, for example, MERQUAT PLUS 3331, available from The Lubrizol Corporation], and the like.

Examples of the quaternized polyvinylpyrrolidone include a quaternary ammonium salt obtained from a copolymer of vinylpyrrolidone (VP) and dimethylaminoethyl methacrylate and diethyl sulfate [polyquaternium-11, for example, GAFQUAT 734 and GAFQUAT 755, all of which are available from ISP Japan Ltd.] and the like.

### (Solubilizing agent)

The solubilizing agent is compounded for the purpose of rending each of the agents of the hair cosmetic material composition liquid. Examples of the solubilizing agent include water, polyhydric alcohols, and organic solvents. Examples of the polyhydric alcohol include glycols and glycerins. Examples of the glycol include ethylene glycol, diethylene glycol, triethylene glycol, propylene glycol, dipropylene glycol, isoprene glycol, 1, 3-butylene glycol, and the like; and examples of the glycerin include glycerin, diglycerin, polyglycerin, and the like. Examples of the organic solvent include ethanol, n-propanol, isopropanol, methyl cellosolve, methyl carbitol, benzyl alcohol, phenethyl alcohol, γ-phenylpropyl alcohol, cinnamic alcohol, p-methylbenzyl alcohol, α-phenylethanol, phenoxyethanol, phenoxyisopropanol, an N-alkylpyrrolidone, an alkylene carbonate, an alkyl ether, and the like. Water is especially preferably used.

### (Water-soluble polymer compound)

As the water-soluble polymer compound, anionic, nonionic, or ampholytic polymer compounds, exclusive of the above-described cationic polymers can be used. Examples thereof include a carboxyvinyl polymer, a diallyl quaternary ammonium salt/acrylic acid copolymer, and the like.

### [Double structure container and hair cosmetic material product]

### (Double structure container)

Next, an example of the double structure container which is used in the embodiment is explained by reference to Fig. 1. In this explanation, portions not related directly to the gist of the present invention are functionally simply explained, and detailed structural explanations thereof are omitted.

An outer container 4 of a double structure container 1 is a pressure-resistant container having such a shape that it is able to stand alone as it is, or by taking a cap (illustration omitted) to be put on a lid as described later as the bottom, in an inverted state and being made of a hard and strong material. Although the outer container 4 may be formed of an opaque metal material, such as stainless steel, etc., it is preferably formed of a plastic material that is hard, strong, and transparent in such a manner that the inside thereof can be seen, and is provided with a needed thickness.

In the inside of the outer container 4, a pouch-shaped first inner container 2 constituting a space for filling the first agent, and a pouch-shaped second inner container 3 constituting a space for filling the second agent are each independently provided. In Fig. 1, on the assumption that the outer container 4 is made of a transparent plastic material, the state in which the pouch-shaped inner containers 2 and 3 in the inside are seen from the outside is illustrated.

The inner containers 2 and 3 are each constituted by using a plastic material different from a constituent material of the outer container 4, the plastic material being comparatively soft so that it is easily deformable by pressure and also being relatively thin and soft. The inner containers 2 and 3 may be each formed in a bag-like body having a laminate structure in consideration of the resistance to breakage. In particular, it is not limited but preferred that the first inner container 2 for filling the first agent containing an alkali agent is made in a laminate structure including a metal layer. Meanwhile, it is not limited but preferred that the second inner container 3 is made transparent or translucent such that the reduced state of the contents in the inner container can be visually recognized.

In the inside of the outer container 4, a space excluding spaces for placing the inner containers 2 and 3 is made as a propellant filling space 9, and a propellant is filled in this space. The propellant is preferably a compressed gas using a nitrogen gas (N₂), carbon dioxide (CO₂), or the like, each of which is inert and low in toxicity, LPG that is a liquefied gas, or the like.

An opening 10 of an upper end of the outer container 4 is airtightly closed by a valve unit 5 that is also a lid. In the inside of the valve unit 5, while illustration is omitted, discharge passages for the first agent and the second agent and valves for closing these discharge passages, respectively are provided. The discharge passages for the first agent and the second agent are connected in a liquid-tight manner to openings of the upper ends of the inner containers 2 and 3, respectively.

It is to be noted that in each of the inner containers 2 and 3, in order to accelerate smooth discharge of the first agent and the second agent to be filled therein, respectively, a rod-like body having a ladder-shaped structure as a whole (illustration omitted; for example, see dip tubes 16A and 16B shown in Fig. 1 of PTL 3) may be inserted from the upper end opening.

As a pair of the valves for opening and closing the discharge passage, a so-called valve stem is adopted in the present embodiment, a pair of cylindrical stems 11 and 12 is protruded in an upper portion of the valve unit 5 and connected in a liquid-tight manner to a pair of discharge passages (illustration omitted) provided in the inside of an actuator 6. These discharge passages in the inside of the actuator 6 are communicated with a pair of discharge holes 7 and 8 provided in an opening 13 of the actuator 6. It is to be noted that the pair of the discharge passages in the inside of the actuator 6 may also be constituted in such a manner that the pair of the discharge passage go into single discharge passage before they reach the opening 13 and are discharged from a single discharge hole of the opening 13.

Meanwhile, the pair of the cylindrical stems 11 and 12 exists at an illustrated position in such a state that it is always pushed upward by a pushing spring (illustration omitted), such as a coil spring built in the valve unit 5, etc. , and at this time, a stem valve is in a "closed" state.

### (Hair cosmetic material product)

The hair cosmetic material product of the present embodiment is one in which the first agent and the second agent of the hair cosmetic material are respectively filled in the inner container 2 and the inner container 3 in the double structure container 1. As the case may be, a third agent is attached as an additional constituent element of the hair cosmetic material product.

In the double structure container 1 having the first agent and the second agent filled therein, both of the first agent and the second agent in the inner containers 2 and 3 always receive a discharge pressure by the propellant for pressurization in the propellant filling space 9. Then, when the actuator 6 is subjected to press-down resisting to a pushing force of the pushing spring, the valve stem becomes in an "open" state, whereby the first agent and the second agent are simultaneously discharged. When a pressing force against the actuator 6 is released, the valve stem becomes in a "closed" state, whereby the discharges of the first agent and the second agent are simultaneously stopped.

### [Examples]

Next, Examples and Comparative Examples of the present invention are explained. It should be construed that the technical scope of the present invention is not limited by the following Examples and Comparative Examples.

### [Preparation of hair cosmetic material]

A first agent and a second agent of each of two-agent type oxidation hair dyeing agents according to Examples 1 to 37 and Comparative Examples 1 to 4, each having a composition shown in the following Tables 1 to 4, respectively, were prepared according to the conventional procedure. All of these first and second agents are in a cream state. In the tables, the numerical value showing the content of each component is a numerical value in terms of a mass% unit in the first agent or the second agent.

Next, the terms "First agent: viscosity" and "Second agent: viscosity" in each of the tables are each a viscosity value (mPa·s) as measured in such a manner that the first agent or the second agent according to each of the Examples or each of the Comparative Examples was stabilized by allowing to stand for 3 days after the preparation, and thereafter, the viscosity was measured by using a BL-type viscometer, VISCOMETER that is a B-type viscometer available from Toki Sangyo Co., Ltd. under measurement conditions of 25°C using a No. 4 rotor for one minute at a rotating rate of 12 rpm/min.

In addition, the terms "First agent: surfactant amount" and "Second agent: surfactant amount" in each of the tables express each a total content (% by mass) of various surfactants in the first agent or the second agent according to each of the Examples and each of the Comparative Examples.

In addition, the terms "First agent: oil amount" and "Second agent: oil amount" in each of the tables each express a total content (% by mass) of various oily components in the first agent or the second agent according to each of the Examples or each of the Comparative Examples.

In addition, the terms "First agent: (carbon number) × (mass)" and "Second agent: (carbon number) × (mass)" in each of the tables each express a total value of the above-described "higher alcohol indexes" in the first agent or the second agent according to each of the Examples or each of the Comparative Examples.

Although not expressed in the tables, all of the second agents according to the respective Examples and Comparative Examples are adjusted to a pH of 3.8.

### [Evaluation of hair cosmetic material]

The hair cosmetic materials according to the respective Examples and respective Comparative Examples were evaluated in the following manner.

### (Degree of consumption of hydrogen peroxide)

A degree of consumption of hydrogen peroxide in the second agent in a state where the second agent of the hair cosmetic material came into contact with the first agent was evaluated by a change of concentration of hydrogen peroxide before and after the contact with the first agent. This degree of consumption hydrogen peroxide is an index of evaluating a generation amount of an oxygen gas in the contact state between the first agent and the second agent.

That is, in a 100-mL tall beaker (available from Hario Co., Ltd., barrel outer diameter: 50 mm, height: 80 mm), 50 g of the first agent immediately after the preparation according to each of the Examples or each of the Comparative Examples was gently poured, and subsequently, 50 g of the second agent immediately after the preparation according to each of the same Examples or each of the same Comparative Examples was gently poured thereonto. At this time, in all of the Examples and Comparative Examples, a two-layer structure including the first agent in a bottom layer and the second agent in a surface layer was formed in the beaker.

Then, for the purpose of avoiding the generation of an error in the measurement concentration to be caused due to evaporation or volatilization of moisture or a volatile component, an upper end opening of the beaker according to each of the Examples or each of the Comparative Examples was immediately hermetically sealed by SARAN WRAP (a registered trademark) and allowed to gentry stand as it was in a cool, dark place for 24 hours. Subsequently, 20 g of the second agent was collected from a portion in a depth of up to 15 mm from the surface in the surface layer (second agent) in the two-layer structure in the beaker according to each of the Examples or each of the Comparative Examples, and after well stirring this, a hydrogen peroxide concentration D1 (%) was measured.

Meanwhile, a hydrogen peroxide concentration D2 (%) in the second agent just before allowing to stand for 24 hours can be accurately determined by calculation because a predetermined amount (% by mass) of hydrogen peroxide is compounded as 35% hydrogen peroxide water in the second agent, and the second agent immediately after the preparation is used. From the foregoing standpoints, the degree of consumption of hydrogen peroxide in the second agent in the contact state of the second agent with the first agent in the hair cosmetic material was calculated as a change of concentration of hydrogen peroxide before and after the contact with the first agent in terms of a subtracted value (%) of (D2 - D1).

The foregoing measurement and calculation of the change of concentration of hydrogen peroxide were performed three times with respect to each of the Examples or each of the Comparative Examples, and an average value thereof was evaluated as the degree of consumption of hydrogen peroxide in the instant Example or Comparative Example. As for evaluation criteria, the case where the subtracted value of (D2 - D1) was 3% or less was evaluated as "⊙"; the case where the subtracted value was more than 3% and 6% or less was evaluated as "○"; the case where the subtracted value was more than 6% and 10% or less was evaluated as "Δ"; and the case where the subtracted value was more than 10% was evaluated as "X". The evaluation results are described in the "Degree of consumption of hydrogen peroxide" row in each of the tables.

### (Uniform mixing properties after discharge)

In preparing the first agent and the second agent according to each of the Examples or each of the Comparative Examples as described above, a coloring agent was previously added in the first agent, and the first agent in a cream state after the preparation was mixed with the second agent in a cream state. As for this mixing operation, the same operation of mixing by stirring with a brush 15 times at the same speed so as to draw a circle was performed commonly in each of the Examples or each of the Comparative Examples. This mixing operation conforms to the usual uniform mixing of the first agent and the second agent.

The presence or absence of color unevenness of the hair cosmetic material according to each of the Examples or each of the Comparative Examples after the above-described mixing operation was evaluated by 10 panelists. As for evaluation criteria, the case where the color unevenness was not observed at all was evaluated as "⊙"; the case where the color unevenness was not substantially observed was evaluated as "○"; the case where the color unevenness was somewhat observed was evaluated as "Δ"; and the case where the color unevenness was significantly observed was evaluated as "×". Then, with respect to the respective Examples and the respective Comparative Examples, the evaluation made by the largest number of the ten panelists was adopted. In the case where there were two or more evaluations made by the largest number of the ten panelists, the lower evaluation was adopted. The evaluation results are described in the "Uniform mixing properties after discharge" row in each of the tables.

### (Brightness)

After preparing the first agent and the second agent in a cream state according to each of the Examples or each of the Comparative Examples, a hair dyeing treatment was performed by uniformly mixing the both agents by using a brush and uniformly applying 2 mL of the mixture to a black hair bundle sample for evaluation having a length of 10 cm, followed by allowing the resultant to stand for 30 minutes. Thereafter, the hair bundle sample was washed with water, dried, and then evaluated for the brightness of hair dyeing by 10 panelists. As for evaluation criteria, the case where the brightness was very good was evaluated as "⊙"; the case where the brightness was good was evaluated as "○"; the case where the brightness was not bad but could not be said to be good was evaluated as "Δ"; and the case where the brightness was bad was evaluated as "×".

In all of the cases, the evaluation made by the largest number of the ten panelists was adopted. In the case where there were two or more evaluations made by the largest number of the ten panelists the lower evaluation was adopted. The evaluation results are described in the "Brightness" row in each of the tables.

**Table 1**

| First agent | Example 1 | Example 2 | Example 3 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|
| Behenyl alcohol | | 3 | | 4 | | | | | | | | | |
| Arachyl alcohol | | | 3 | | 4 | | | | | | | | |
| Stearyl stearate | 3 | | | 3 | 3 | 3 | 3 | 3 | 3 | 3 | 3 | 3 | 3 |
| Cetyl alcohol | 4 | 4 | 4 | | | 4 | 4 | 4 | 4 | 4 | 4 | 4 | 4 |
| POE(30) cetyl ether | 2 | 2 | 2 | 2 | 2 | | 2 | 2 | 2 | 2 | | 2 | 2 |
| POE(20) stearyl ether | | | | | | 2 | | | | | | | |
| POE(2) cetyl ether | 1 | 1 | 1 | 1 | 1 | 1 | | 1 | 1 | | 1 | 1 | 1 |
| POE(2) lauryl ether | | | | | | | 1 | | | | | | |
| Glyceryl stearate | | | | | | | | | | 1 | | | |
| Alkyl alucoside | | | | | | | | | | | 2 | | |
| Stearvltrimethylammonium chloride | 1 | 1 | 1 | 1 | 1 | 1 | 1 | 1 | 1 | 1 | 1 | 1 | 1 |
| Sodium laureth sulfate | | | | | | | | | | | | | |
| Cocamidopropyl betaine | | | | | | | | | | | | | |
| Sodium chloride | | | | | | | | | | | | | |
| Hydroxyethyl cellulose | | | | | | | | | | | | | |
| Vaseline | 3 | 3 | 3 | 3 | 3 | 3 | 3 | | | 3 | 3 | 3 | 3 |
| Cetyl octanoate | | | | | | | | 1 | | | | | |
| Lanolin | | | | | | | | | 2 | | | | |
| Stearyl stearate | | | | | | | | | 1 | | | | |
| Microcrystalline wax | | | | | | | | 2 | | | | | |
| Sodium sulfite | 0.1 | 0.1 | 0.1 | 0.1 | 0.1 | 0.1 | 0.1 | 0.1 | 0.1 | 0.1 | 0.1 | 0.1 | 0.1 |
| p-Phenylenediamine | 0.5 | 0.5 | 0.5 | 0.5 | 0.5 | 0.5 | 0.5 | 0.5 | 0.5 | 0.5 | 0.5 | 0.5 | 0.5 |
| m-Aminophenol | 0.4 | 0.4 | 0.4 | 0.4 | 0.4 | 0.4 | 0.4 | 0.4 | 0.4 | 0.4 | 0.4 | 0.4 | 0.4 |
| Resorcin | 0.5 | 0.5 | 0.5 | 0.5 | 0.5 | 0.5 | 0.5 | 0.5 | 0.5 | 0.5 | 0.5 | 0.5 | 0.5 |
| Ascorbic acid | 0.2 | 0.2 | 0.2 | 0.2 | 0.2 | 0.2 | 0.2 | 0.2 | 0.2 | 0.2 | 0.2 | 0.2 | 0.2 |
| 28% ammonia water | 4 | 4 | 4 | 4 | 4 | 4 | 4 | 4 | 4 | 4 | 4 | 4 | 4 |
| Purified water | Proper amount | Proper amount | Proper amount | Proper amount | Proper amount | Proper amount | Proper amount | Proper amount | Proper amount | Proper amount | Proper amount | Proper amount | Proper amount |
| Total | 100 | 100 | 100 | 100 | 100 | 100 | 100 | 100 | 100 | 100 | 100 | 100 | 100 |
| | | | | | | | | | | | | | |

| Second agent | | | | | | | | | | | | | |
|---|---|---|---|---|---|---|---|---|---|---|---|---|---|
| Stearyl alcohol | 1 | 1 | 1 | 1 | 1 | 1 | 1 | 1 | 1 | 1 | 1 | 1 | 1 |
| Cetyl alcohol | 4 | 4 | 4 | 4 | 4 | 4 | 4 | 4 | 4 | 4 | 4 | 4 | 4 |
| POE(30) cetyl ether | 1 | 1 | 1 | 1 | 1 | 1 | 1 | 1 | 1 | 1 | 1 | 1 | |
| POE(2) cetyl ether | 0.5 | 0.5 | 0.5 | 0.5 | 0.5 | 0.5 | 0.5 | 0.5 | 0.5 | 0.5 | 0.5 | | 0.5 |
| Glvceryl stearate | | | | | | | | | | | | 0.5 | |
| Alkyl glucoside | | | | | | | | | | | | | 1 |
| Stearyltrimethylammonium chloride | 1 | 1 | 1 | 1 | 1 | 1 | 1 | 1 | 1 | 1 | 1 | 1 | 1 |
| Sodium chloride | | | | | | | | | | | | | |
| Hydroxyethyl cellulose | | | | | | | | | | | | | |
| Vaseline | 2 | 2 | 2 | 2 | 2 | 2 | 2 | 2 | 2 | 2 | 2 | 2 | 2 |
| Microcrystalline wax | | | | | | | | | | | | | |
| Phenoxyethanol | 0.2 | 0.2 | 0.2 | 0.2 | 0.2 | 0.2 | 0.2 | 0.2 | 0.2 | 0.2 | 0.2 | 0.2 | 0.2 |
| Hvdroxyethanediphosphonic acid | 0.1 | 0.1 | 0.1 | 0.1 | 0.1 | 0.1 | 0.1 | 0.1 | 0.1 | 0.1 | 0.1 | 0.1 | 0.1 |
| Tetrasodium hydroxyethanediphosphonate | 0.1 | 0.1 | 0.1 | 0.1 | 0.1 | 0.1 | 0.1 | 0.1 | 0.1 | 0.1 | 0.1 | 0.1 | 0.1 |
| 35% hydrogen peroxide | 16 | 16 | 16 | 16 | 16 | 16 | 16 | 16 | 16 | 16 | 16 | 16 | 16 |
| Purified water | Proper amount | Proper amount | Proper amount | Proper amount | Proper amount | Proper amount | Proper amount | Proper amount | Proper amount | Proper amount | Proper amount | Proper amount | Proper amount |
| Total | 100 | 100 | 100 | 100 | 100 | 100 | 100 | 100 | 100 | 100 | 100 | 100 | 100 |
| First agent: viscosity | 18580 | 13640 | 11210 | 20870 | 10720 | 16320 | 19850 | 20100 | 16890 | 27410 | 7100 | 18580 | 7100 |
| Second agent: viscosity | 12650 | 12650 | 12650 | 12650 | 12650 | 12650 | 12650 | 12650 | 12650 | 12650 | 12650 | 19490 | 12650 |
| First agent: surfactant amount | 4 | 4 | 4 | 4 | 4 | 4 | 4 | 4 | 4 | 4 | 4 | 4 | 4 |
| Second agent: surfactant amount | 2.5 | 2.5 | 2.5 | 2.5 | 2.5 | 2.5 | 2.5 | 2.5 | 2.5 | 2.5 | 2.5 | 2.5 | 2.5 |
| First agent: oil amount | 3 | 3 | 3 | 3 | 3 | 3 | 3 | 3 | 3 | 3 | 3 | 3 | 3 |
| Second agent: oil amount | 2 | 2 | 2 | 2 | 2 | 2 | 2 | 2 | 2 | 2 | 2 | 2 | 2 |
| First agent: (carbon number) × (mass) | 118 | 130 | 124 | 142 | 134 | 118 | 118 | 118 | 118 | 118 | 118 | 118 | 118 |
| Second agent: (carbon number) × (mass) | 82 | 82 | 82 | 82 | 82 | 82 | 82 | 82 | 82 | 82 | 82 | 82 | 82 |
| Degree of consumption of hydrogen peroxide | ⊚ | ⊚ | ⊚ | ⊚ | ⊚ | ⊚ | ⊚ | ⊚ | ⊚ | ⊚ | ⊚ | ⊚ | ⊚ |
| Uniform mixing properties after discharge | ⊚ | ⊚ | ⊚ | ⊚ | ⊚ | ⊚ | ⊚ | ⊚ | ⊚ | ⊚ | ⊚ | ⊚ | ⊚ |
| Brightness | ⊚ | ⊚ | ⊚ | ⊚ | ⊚ | ⊚ | ⊚ | ⊚ | ⊚ | ⊚ | ⊚ | ⊚ | ⊚ |

**Table 2**

| First agent | Example 14 | Example 15 | Example 16 | Example 17 | Example 18 | Example 19 | Example 20 | Example 21 | Example 22 | Example 23 |
|---|---|---|---|---|---|---|---|---|---|---|
| Behenyl alcohol | | | | | | | | | | |
| Arachyl alcohol | | | | | | | | | | |
| Stearyl alcohol | 3 | 3 | 3 | 3 | 3 | 3 | 3 | 3 | 3 | 3 |
| Cetyl alcohol | 4 | 4 | 4 | 4 | 4 | 4 | 4 | 4 | 4 | 4 |
| POE(30) cetyl ether | 2 | 2 | 2 | 2 | 2 | 2 | 2 | 2 | 2 | 2 |
| POE(2) cetyl ether | 1 | 1 | 1 | 1 | 1 | 1 | 1 | 1 | 1 | 1 |
| Stearyltrimethylammonium chloride | 1 | 1 | 1 | 1 | 1 | 1 | 1 | 1 | 1 | 1 |
| Sodium laureth sulfate | | | | | | | | | | |
| Cocamidopropyl betaine | | | | | | | | | | |
| Sodium chloride | 6 | 4 | | | | | | | 6 | |
| Hydroxyethyl cellulose | | | 0.5 | 2 | | | | | | 2 |
| Vaseline | 3 | 3 | 3 | 3 | 3 | 3 | 3 | 3 | 3 | 3 |
| Microcrystalline wax | | | | | | | | | | |
| Sodium sulfite | 0.1 | 0.1 | 0.1 | 0.1 | 0.1 | 0.1 | 0.1 | 0.1 | 0.1 | 0.1 |
| p-Phenylenediamine | 0.5 | 0.5 | 0.5 | 0.5 | 0.5 | 0.5 | 0.5 | 0.5 | 0.5 | 0.5 |
| m-Aminophenol | 0.4 | 0.4 | 0.4 | 0.4 | 0.4 | 0.4 | 0.4 | 0.4 | 0.4 | 0.4 |
| Resorcin | 0.5 | 0.5 | 0.5 | 0.5 | 0.5 | 0.5 | 0.5 | 0.5 | 0.5 | 0.5 |
| Ascorbic acid | 0.2 | 0.2 | 0.2 | 0.2 | 0.2 | 0.2 | 0.2 | 0.2 | 0.2 | 0.2 |
| 28% ammonia water | 4 | 4 | 4 | 4 | 4 | 4 | 4 | 4 | 4 | 4 |
| Purified water | Proper amount | Proper amount | Proper amount | Proper amount | Proper amount | Proper amount | Proper amount | Proper amount | Proper amount | Proper amount |
| Total | 100 | 100 | 100 | 100 | 100 | 100 | 100 | 100 | 100 | 100 |
| | | | | | | | | | | |

| Second agent | | | | | | | | | | |
|---|---|---|---|---|---|---|---|---|---|---|
| Stearyl alcohol | 1 | 1 | 1 | 1 | 1 | 1 | 1 | 1 | 1 | 1 |
| Cetyl alcohol | 4 | 4 | 4 | 4 | 4 | 4 | 4 | 4 | 4 | 4 |
| POH(30) cetyl ether | 1 | 1 | 1 | 1 | 1 | 1 | 1 | 1 | 1 | 1 |
| POE(2) cetyl ether | 0.5 | 0.5 | 0.5 | 0.5 | 0.5 | 0.5 | 0.5 | 0.5 | 0.5 | 0.5 |
| Stearyltrimethylammonium chloride | 1 | 1 | 1 | 1 | 1 | 1 | 1 | 1 | 1 | 1 |
| Sodium chloride | | | | | 2 | 0.5 | | | 2 | |
| Hydroxyethyl cellulose | | | | | | | 1 | 2.5 | | 2.5 |
| Vaseline | 2 | 2 | 2 | 2 | 2 | 2 | 2 | 2 | 2 | 2 |
| Microcrystalline wax | | | | | | | | | | |
| Phenoxyethanol | 0.2 | 0.2 | 0.2 | 0.2 | 0.2 | 0.2 | 0.2 | 0.2 | 0.2 | 0.2 |
| Hvdroxyethanediphosphonic acid | 0.1 | 0.1 | 0.1 | 0.1 | 0.1 | 0.1 | 0.1 | 0.1 | 0.1 | 0.1 |
| Tetrasodium hydroxyethanediphosphonate | 0.1 | 0.1 | 0.1 | 0.1 | 0.1 | 0.1 | 0.1 | 0.1 | 0.1 | 0.1 |
| 35% hydrogen peroxide | 16 | 16 | 16 | 16 | 16 | 16 | 16 | 16 | 16 | 16 |
| Purified water | Proper amount | Proper amount | Proper amount | Proper amount | Proper amount | Proper amount | Proper amount | Proper amount | Proper amount | Proper amount |
| Total | 100 | 100 | 100 | 100 | 100 | 100 | 100 | 100 | 100 | 100 |
| First agent: viscosity | 7200 | 10540 | 20600 | 29600 | 18580 | 18580 | 18580 | 18580 | 7200 | 29600 |
| Second agent: viscosity | 12650 | 12650 | 12650 | 12650 | 7400 | 10520 | 19850 | 28900 | 7400 | 28900 |
| First agent: surfactant amount | 4 | 4 | 4 | 4 | 4 | 4 | 4 | 4 | 4 | 4 |
| Second agent: surfactant amount | 2.5 | 2.5 | 2.5 | 2.5 | 2.5 | 2.5 | 2.5 | 2.5 | 2.5 | 2.5 |
| First agent: oil amount | 3 | 3 | 3 | 3 | 3 | 3 | 3 | 3 | 3 | 3 |
| Second agent: oil amount | 2 | 2 | 2 | 2 | 2 | 2 | 2 | 2 | 2 | 2 |
| First agent: (carbon number) × (mass) | 118 | 118 | 118 | 118 | 118 | 118 | 118 | 118 | 118 | 118 |
| Second agent: (carbon number) × (mass) | 82 | 82 | 82 | 82 | 82 | 82 | 82 | 82 | 82 | 82 |
| Degree of consumption of hydrogen peroxide | ⊚ | ⊚ | ⊚ | ⊚ | ⊚ | ⊚ | ⊚ | ⊚ | ⊚ | ⊚ |
| Uniform mixing properties after discharge | ⊚ | ⊚ | ⊚ | ⊚ | ⊚ | ⊚ | ⊚ | ⊚ | ⊚ | ⊚ |
| Brightness | ⊚ | ⊚ | ⊚ | ⊚ | ⊚ | ⊚ | ⊚ | ⊚ | ⊚ | ⊚ |

**Table 3**

| First agent | Example 24 | Example 25 | Example 26 | Example 27 | Example 28 | Example 29 | Example 30 | Example 31 | Example 32 | Example 33 | Example 34 | Example 35 | Example 36 | Example 37 |
|---|---|---|---|---|---|---|---|---|---|---|---|---|---|---|
| Stearyl alcohol | 3 | 3 | 3 | 3 | 3 | 3 | | 3 | 4 | 3 | 3 | 6 | 3 | 3 |
| Cetyl alcohol | 4 | 4 | 4 | 4 | 4 | 4 | 3 | 1 | 4 | 4 | 4 | 1 | 4 | 4 |
| POH(30) cetyl ether | 0.8 | 1.4 | 4 | 5 | 2 | 2 | 2 | 2 | 2 | 2 | 2 | 2 | 2 | 2 |
| POE(2) cetyl ether | 0.4 | 0.7 | 2 | 2.5 | 1 | 1 | 1 | 1 | 1 | 1 | 1 | 1 | 1 | 1 |
| Stearyltrimethylammonium chloride | 0.4 | 0.7 | 2 | 2.5 | 1 | 1 | 1 | 1 | 1 | 1 | 1 | 1 | 1 | 0.5 |
| Sodium lauryl sulfate | | | | | | | | | | | | | | 0.5 |
| Vaseline | 3 | 3 | 3 | 3 | 1 | 6 | 3 | 3 | 3 | 3 | 7 | 3 | 3 | 3 |
| Stearyl stearate | | | | | | | 3 | | | | | | | |
| Sodium sulfite | 0.1 | 0.1 | 0.1 | 0.1 | 0.1 | 0.1 | 0.1 | 0.1 | 0.1 | 0.1 | 0.1 | 0.1 | 0.1 | 0.1 |
| p-Phenylenediamine | 0.5 | 0.5 | 0.5 | 0.5 | 0.5 | 0.5 | 0.5 | 0.5 | 0.5 | 0.5 | 0.5 | 0.5 | 0.5 | 0.5 |
| m-Aminophenol | 0.4 | 0.4 | 0.4 | 0.4 | 0.4 | 0.4 | 0.4 | 0.4 | 0.4 | 0.4 | 0.4 | 0.4 | 0.4 | 0.4 |
| Resorcin | 0.5 | 0.5 | 0.5 | 0.5 | 0.5 | 0.5 | 0.5 | 0.5 | 0.5 | 0.5 | 0.5 | 0.5 | 0.5 | 0.5 |
| Ascorbic acid | 0.2 | 0.2 | 0.2 | 0.2 | 0.2 | 0.2 | 0.2 | 0.2 | 0.2 | 0.2 | 0.2 | 0.2 | 0.2 | 0.2 |
| 28% ammonia water | 4 | 4 | 4 | 4 | 4 | 4 | 4 | 4 | 4 | 4 | 4 | 4 | 4 | 4 |
| Purified water | Proper amount | Proper amount | Proper amount | Proper amount | Proper amount | Proper amount | Proper amount | Proper amount | Proper amount | Proper amount | Proper amount | Proper amount | Proper amount | Proper amount |
| Total | 100 | 100 | 100 | 100 | 100 | 100 | 100 | 100 | 100 | 100 | 100 | 100 | 100 | 100 |
| | | | | | | | | | | | | | | |

| Second agent | | | | | | | | | | | | | | |
|---|---|---|---|---|---|---|---|---|---|---|---|---|---|---|
| Stearyl alcohol | 1 | 1 | 1 | 1 | 1 | 1 | 1 | 1 | 1 | 1 | 1 | 1 | 1 | 1 |
| Cetyl alcohol | 4 | 4 | 4 | 4 | 4 | 4 | 4 | 4 | 4 | 4 | 4 | 4 | 4 | 4 |
| POE(30) cetyl ether | 1 | 1 | 1 | 1 | 1 | 1 | 1 | 1 | 1 | 1 | 1 | 1 | 1 | 1 |
| POE(2) cetyl ether | 0.5 | 0.5 | 0.5 | 0.5 | 0.5 | 0.5 | 0.5 | 0.5 | 0.5 | 0.5 | 0.5 | 0.5 | 0.5 | 0.5 |
| Stearyltrimethylammonium chloride | 1 | 1 | 1 | 1 | 1 | 1 | 1 | 1 | 1 | 1 | 1 | 1 | 0.5 | 1 |
| Sodium lauryl sulfate | | | | | | | | | | | | | 0.5 | |
| Vaseline | 2 | 2 | 2 | 2 | 0.5 | 3 | 2 | 2 | 2 | 2.8 | 1.5 | 2 | 2 | 2 |
| Phenoxyethanol | 0.2 | 0.2 | 0.2 | 0.2 | 0.2 | 0.2 | 0.2 | 0.2 | 0.2 | 0.2 | 0.2 | 0.2 | 0.2 | 0.2 |
| Hvdroxyethanediphosphonic acid | 0.1 | 0.1 | 0.1 | 0.1 | 0.1 | 0.1 | 0.1 | 0.1 | 0.1 | 0.1 | 0.1 | 0.1 | 0.1 | 0.1 |
| Tetrasodium hydroxyethanediphosphonate | 0.1 | 0.1 | 0.1 | 0.1 | 0.1 | 0.1 | 0.1 | 0.1 | 0.1 | 0.1 | 0.1 | 0.1 | 0.1 | 0.1 |
| 35% hydrogen peroxide | 16 | 16 | 16 | 16 | 16 | 16 | 16 | 16 | 16 | 16 | 16 | 16 | 16 | 16 |
| Purified water | Proper amount | Proper amount | Proper amount | Proper amount | Proper amount | Proper amount | Proper amount | Proper amount | Proper amount | Proper amount | Proper amount | Proper amount | Proper amount | Proper amount |
| Total | 100 | 100 | 100 | 100 | 100 | 100 | 100 | 100 | 100 | 100 | 100 | 100 | 100 | 100 |
| First agent: viscosity | 9800 | 8450 | 25600 | 26500 | 17800 | 17500 | 14200 | 13200 | 20850 | 18580 | 16350 | 21900 | 18580 | 16390 |
| Second agent: viscosity | 12650 | 12650 | 12650 | 12650 | 12900 | 12320 | 12650 | 12650 | 12650 | 12500 | 12650 | 12650 | 9800 | 12650 |
| First agent: surfactant amount | 1.6 | 2.8 | 8 | 10 | 4 | 4 | 4 | 4 | 4 | 4 | 4 | 4 | 4 | 4 |
| Second agent: surfactant amount | 2.5 | 2.5 | 2.5 | 2.5 | 2.5 | 2.5 | 2.5 | 2.5 | 2.5 | 2.5 | 2.5 | 2.5 | 2.5 | 2.5 |
| First agent: oil amount | 3 | 3 | 3 | 3 | 1 | 6 | 6 | 3 | 3 | 3 | 7 | 3 | 3 | 3 |
| Second agent: oil amount | 2 | 2 | 2 | 2 | 0.5 | 3 | 2 | 2 | 2 | 2.8 | 1.5 | 2 | 2.5 | 2 |
| First agent: (carbon number) × (mass) | 118 | 118 | 118 | 118 | 118 | 118 | 48 | 70 | 136 | 118 | 118 | 124 | 118 | 118 |
| Second agent: (carbon number) × (mass) | 82 | 82 | 82 | 82 | 82 | 82 | 82 | 82 | 82 | 82 | 82 | 82 | 82 | 82 |
| Degree of consumption of hydrogen peroxide | ⊚ | ⊚ | ⊚ | ⊚ | ⊚ | ⊚ | ⊚ | ⊚ | ⊚ | ⊚ | ⊚ | ⊚ | ⊚ | ⊚ |
| Uniform mixing properties after discharge | ⊚ | ⊚ | ⊚ | ⊚ | ⊚ | ⊚ | ⊚ | ⊚ | ⊚ | ⊚ | ⊚ | ⊚ | ⊚ | ⊚ |
| Brightness | ⊚ | ⊚ | ⊚ | ○ | ○ | ⊚ | ⊚ | ⊚ | ⊚ | ⊚ | ⊚ | ⊚ | ⊚ | ⊚ |

**Table 4**

| First agent | Comparative Example 1 | Comparative Example 2 | Comparative Example 3 | Comparative Example 4 |
|---|---|---|---|---|
| Behenyl alcohol | | | | |
| Arachyl alcohol | | | | |
| Stearyl alcohol | 3 | 3 | 3 | 3 |
| Cetyl alcohol | 4 | 4 | 4 | 4 |
| POE(30) cetyl ether | 2 | 2 | 2 | 2 |
| POE(2) cetyl ether | 1 | 1 | 1 | 1 |
| Stearyltrimthylammonium chloride | 1 | 1 | 1 | 1 |
| Sodium laureth sulfate | | | | |
| Cocamidopropyl betaine | | | | |
| Sodium chloride | 8 | | | |
| Hydroxyethyl cellulose | | 3 | | |
| Vaseline | 3 | 3 | 3 | 3 |
| Microcrystalline wax | | | | |
| Sodium sulfite | 0.1 | 0.1 | 0.1 | 0.1 |
| p-Phenylenediamine | 0.5 | 0.5 | 0.5 | 0.5 |
| m-Aminophenol | 0.4 | 0.4 | 0.4 | 0.4 |
| Resorcin | 0.5 | 0.5 | 0.5 | 0.5 |
| Ascorbic acid | 0.2 | 0.2 | 0.2 | 0.2 |
| 28% ammonia water | 4 | 4 | 4 | 4 |
| Purified water | Proper amount | Proper amount | Proper amount | Proper amount |
| Total | 100 | 100 | 100 | 100 |
| | | | | |

| Second agent | | | | |
|---|---|---|---|---|
| Stearyl alcohol | 1 | 1 | 1 | 1 |
| Cetyl alcohol | 4 | 4 | 4 | 4 |
| POE(30) cetyl ether | 1 | 1 | 1 | 1 |
| POE(2) cetyl ether | 0.5 | 0.5 | 0.5 | 0.5 |
| Stearyltrimethylammonium chloride | 1 | 1 | 1 | 1 |
| Sodium chloride | | | 4 | |
| Hydroxyethyl cellulose | | | | 4 |
| Vaseline | 2 | 2 | 2 | 2 |
| Microcrystalline wax | | | | |
| Phenoxyethanol | 0.2 | 0.2 | 0.2 | 0.2 |
| Hydroxyethanediphosphonic acid | 0.1 | 0.1 | 0.1 | 0.1 |
| Tetrasodium hydroxyethanediphosphonate | 0.1 | 0.1 | 0.1 | 0.1 |
| 35% hydrogen peroxide | 16 | 16 | 16 | 16 |
| Purified water | Proper amount | Proper amount | Proper amount | Proper amount |
| Total | 100 | 100 | 100 | 100 |
| First agent: viscosity | 5200 | 35000 | 18580 | 18580 |
| Second agent: viscosity | 12650 | 12650 | 4800 | 35800 |
| First agent: surfactant amount | 4 | 4 | 4 | 4 |
| Second agent: surfactant amount | 2.5 | 2.5 | 2.5 | 2.5 |
| First agent: oil amount | 3 | 3 | 3 | 3 |
| Second agent: oil amount | 2 | 2 | 2 | 2 |
| First agent: (carbon number) × (mass) | 118 | 118 | 118 | 118 |
| Second agent: (carbon number) × (mass) | 82 | 82 | 82 | 82 |
| Degree of consumption of hydrogen peroxide | × | × | × | × |
| Uniform mixing properties after discharge | ○ | ○ | ○ | ○ |
| Brightness | ⊚ | ⊚ | ⊚ | ⊚ |

According to the present invention, a hair cosmetic material in which the generation of an oxygen gas to be caused due to contact between a first agent and a second agent, each of which has leaked into the inside of an outer container from a bag-like body in a double structure container, is reduced is provided.

## Claims

1. A hair cosmetic material comprising a first agent containing an alkali agent and a second agent containing an oxidizing agent, wherein
the first agent and the second agent are used in a double structure container in which a space for filling the first agent and a space for filling the second agent are each independently provided in the inside of a compressed gas filling space having a propellant for pressurization filled therein, and which is provided with a mechanism of separating the first agent and the second agent, which are each filled in the space for filling the respective agents, from each other and simultaneously discharging the both agents by the propellant; and
each of the first agent and the second agent is discharged in a liquid state, the first agent contains an alkali agent, whereas the second agent contains an oxidizing agent, and each of the first agent and the second agent has a viscosity falling within the range of from 7,000 to 30,000 mPa·s at 25°C as measured, for example, by using a B-type viscometer for one minute at a rotating rate of 12 rpm/min under conditions of using a No. 4 rotor.

2. The hair cosmetic material according to claim 1, wherein each of the first agent and the second agent contains at least one surfactant, and a content of the surfactant of each of the agents is 10% by mass or less.

3. The hair cosmetic material according to claim 1 or 2, wherein each of the first agent and the second agent contains at least one oily component, and a total content of the oily components in the first agent and the second agent relative to a total amount of the first agent and the second agent is 10% by mass or less.

4. The hair cosmetic material according to any one of claims 1 to 3, wherein each of the first agent and the second agent contains at least one alcohol having a carbon number in the range of from 12 to 22, and a total value of the following alcohol indexes regarding the alcohol contained in each of the agents is 140 or less:
alcohol index: an integrated value (a × b) of a carbon number (a) of the alcohol and a content value (b) in the first agent or the second agent of the alcohol in terms of a mass% unit.

5. A hair cosmetic material product comprising
the hair cosmetic material according to any one of claims 1 to 4; and
a double structure container in which a space for filling the first agent and a space for filling the second agent are each independently provided in the inside of a propellant filling space having a propellant for pressurization filled therein, and which is provided with a mechanism of separating the first agent and the second agent to be filled in the spaces for filling the respective agents from each other and simultaneously discharging the both agents by the propellant, wherein
the first agent and the second agent of the hair cosmetic material are filled in the space for filling the first agent and the space for filling the second agent, respectively in the double structure container.

## Patentansprüche

1. Haarkosmetikmaterial, das ein erstes Mittel, das ein Alkalisierungsmittel enthält, und ein zweites Mittel umfasst, das ein Oxidationsmittel enthält, wobei
das erste Mittel und das zweite Mittel in einem Doppelstrukturbehälter verwendet werden, in welchem ein Raum für die Füllung des ersten Mittels und ein Raum für die Füllung des zweiten Mittels jeweils unabhängig im Inneren eines Kompressionsgasfüllraums bereitgestellt werden, der ein Treibmittel für den Druckaufbau darin eingefüllt enthält, und welcher mit einem Mechanismus für die Trennung des ersten und des zweiten Mittels voneinander, welche jeweils in dem Raum für das Füllen der entsprechenden Mittel gefüllt sind, und gleichzeitiges Entladen der beiden Mittel durch das Treibmittel, versehen ist; und
jedes des ersten Mittels und des zweiten Mittels in einem flüssigen Zustand entladen wird, das erste Mittel ein Alkalisierungsmittel enthält, während das zweite Mittel ein Oxidationsmittel enthält, und jedes des ersten Mittels und des zweiten Mittels eine Viskosität aufweist, die bei 25°C in einem Bereich von 7 000 bis 30 000 mPa·s fällt, gemessen durch, zum Beispiel, Verwendung eines Viskosimeters vom B-Typ für eine Minute bei einer Drehungsgeschwindigkeit von 12 U/min unter Bedingung der Verwendung eines Rotors Nr. 4.

2. Haarkosmetikmaterial nach Anspruch 1, wobei jedes des ersten Mittels und des zweiten Mittels wenigstens einen oberflächenaktiven Stoff enthält, und ein Gehalt des oberflächenaktiven Stoffs von jedem der Mittel 10 Massen-% oder weniger ist.

3. Haarkosmetikmaterial nach Anspruch 1 oder 2, wobei jedes des ersten Mittels und des zweiten Mittels wenigstens einen öligen Bestandteil enthält, und ein Gesamtgehalt der öligen Bestandteile in dem ersten Mittel und dem zweiten Mittel relativ zu der Gesamtmenge des ersten Mittels und des zweiten Mittels 10 Massen-% oder weniger ist.

4. Haarkosmetikmaterial nach einem der Ansprüche 1 bis 3, wobei jedes des ersten Mittels und des zweiten Mittels wenigstens einen Alkohol enthält, der eine Kohlenstoffzahl in dem Bereich von 12 bis 22 aufweist, und ein Gesamtwert der folgenden Alkoholindizes bezogen auf den in jedem der Mittel enthaltenen Alkohol 140 oder weniger ist:
Alkoholindex: ein integrierter Wert (a × b) einer Kohlenstoffzahl (a) des Alkohols und eines Gehaltwertes (b) in dem ersten Mittel oder dem zweiten Mittel des Alkohols in Bezug auf eine Massen-%-Einheit.

5. Haarkosmetikmaterialprodukt, das umfasst
das Haarkosmetikmaterial nach einem der Ansprüche 1 bis 4;
einen Doppelstrukturbehälter, in welchem ein Raum für die Füllung des ersten Mittels und ein Raum für die Füllung des zweiten Mittels jeweils unabhängig im Inneren eines Kompressionsgasfüllraums bereitgestellt werden, der ein Treibmittel für den Druckaufbau darin eingefüllt enthält, und welcher mit einem Mechanismus für die Trennung des ersten und des zweiten Mittels voneinander, welche jeweils in dem Raum für das Füllen der entsprechenden Mittel gefüllt sind, und gleichzeitiges Entladen der beiden Mittel durch das Treibmittel, versehen ist, wobei
das erste Mittel und das zweite Mittel des Haarkosmetikmaterials in den Raum für das Füllen des ersten Mittels bzw. den Raum für das Füllen des zweiten Mittels in dem Doppelstrukturbehälter gefüllt sind.

## Revendications

1. Matériau cosmétique capillaire comprenant un premier agent contenant un agent alcalin et un deuxième agent contenant un agent oxydant, dans lequel
le premier agent et le deuxième agent sont utilisés dans un récipient à structure double dans lequel un espace devant être rempli du premier agent et un espace devant être rempli du deuxième agent sont chacun indépendamment disposés à l'intérieur d'un espace de remplissage par du gaz comprimé ayant un propulseur pour pressurisation qui le remplit, et qui est doté d'un mécanisme de séparation l'un de l'autre du premier agent et du deuxième agent, qui remplissent chacun l'espace devant être rempli par les agents respectifs, et simultanément de décharge des deux agents par le propulseur ; et
chacun du premier agent et du deuxième agent est déchargé à l'état liquide, le premier agent contient un agent alcalin, tandis que le deuxième agent contient un agent oxydant, et chacun du premier agent et du deuxième agent a une viscosité située dans la plage allant de 7 000 à 30 000 mPa.s à 25°C, telle que mesurée par exemple au moyen d'un viscosimètre de type B pendant une minute à une vitesse de rotation de 12 t/min dans des conditions d'utilisation d'un rotor N° 4.

2. Matériau cosmétique capillaire selon la revendication 1, dans lequel chacun du premier agent et du deuxième agent contient au moins un tensioactif, et la teneur en le tensioactif de chacun des agents est de 10 % en masse ou moins.

3. Matériau cosmétique capillaire selon la revendication 1 ou 2, dans lequel chacun du premier agent et du deuxième agent contient au moins un composant huileux, et la teneur totale en les composants huileux du premier agent et du deuxième agent par rapport à la quantité totale du premier agent et du deuxième agent est de 10 % en masse ou moins.

4. Matériau cosmétique capillaire selon l'une quelconque des revendications 1 à 3, dans lequel chacun du premier agent et du deuxième agent contient au moins un alcool ayant de 12 à 22 carbones, et la valeur totale des indices d'alcool suivants, concernant l'alcool contenu dans chacun des agents, est de 140 ou moins :
indice d'alcool : la valeur intégrée (a x b) du nombre de carbones (a) de l'alcool et de la valeur en contenu (b) dans le premier agent ou le deuxième agent de l'alcool en termes d'une unité de % en masse.

5. Produit de matériau cosmétique capillaire comprenant
un matériau cosmétique capillaire selon l'une quelconque des revendications 1 à 4 ; et
un récipient à structure double dans lequel un espace devant être rempli du premier agent et un espace devant être rempli du deuxième agent sont chacun indépendamment disposés à l'intérieur d'un espace de remplissage par un propulseur ayant un propulseur pour pressurisation qui le remplit, et qui est doté d'un mécanisme de séparation l'un de l'autre du premier agent et du deuxième agent devant remplir les espaces destinés à être remplis par les agents respectifs, et simultanément de décharge des deux agents par le propulseur, dans lequel
le premier agent et le deuxième agent du matériau cosmétique capillaire remplissent l'espace devant être rempli par le premier agent et l'espace devant être rempli par le deuxième agent, respectivement, dans le récipient à structure double.
